# FASCICULE DE BREVET EUROPEEN

(11) **EP 0 761 306 B1**
(45) Date de publication et mention de la délivrance du brevet: **16.05.2001**
(21) Numéro de dépôt: 96401896.4
(22) Date de dépôt: 05.09.1996
(51) Int. Cl.: B01J 31/02, C07C 2/62

(54) **Catalyseur solide d'alkylation aliphatique**
Fester Katalysator für aliphatische Alkylierung
Solid catalyst for aliphatic alkylation

(30) Priorité: 11.09.1995 FR 9510695
(43) Date de publication de la demande: 12.03.1997
(73) Titulaire: INSTITUT FRANCAIS DU PETROLE, 92502 Rueil-Malmaison (FR)
(72) Inventeur: Benazzi, Eric, 78360 Montesson (FR); Joly, Jean-François, 69006 Lyon (FR)

(56) Documents cités:
- EP-A- 0 605 279
- EP-A- 0 623 388
- US-A- 5 349 116

## Description

La présente invention concerne un catalyseur comprenant un support poreux organique ou minéral et au moins une phase active comprenant au moins un acide choisi parmi les acides de formule R-SO₃H, où R est le fluor ou un groupement alkyl ou un groupement alkyl fluoré (partiellement ou totalement fluoré), R étant de préférence F ou CF₃, de façon encore plus préférée CF₃, et au moins un solvant aprotique organique faiblement basique. L'invention concerne aussi l'utilisation en alkylation dite aliphatique dudit catalyseur, c'est-à-dire en alkylation catalytique d'isobutane et/ou d'isopentane au moyen d'au moins une oléfine comprenant de 3 à 6 atomes de carbone par molécule (c'est-à-dire d'une oléfine C₃-C₆), ce qui permet d'obtenir des composés paraffiniques à haut degré de ramification et à haut indice d'octane.

Un grand nombre de catalyseurs acides, liquides ou solides, sont connus pour réaliser l'alkylation aliphatique d'isoparaffine(s) telles que l'isobutane ou l'isopentane, par au moins une oléfine telle que le propylène, les butènes-1 et -2 ou l'isobutène. Les catalyseurs les plus couramment utilisés dans la pratique industrielle sont les catalyseurs liquides que sont l'acide sulfurique concentré et l'acide fluorhydrique seul ou en mélange avec des acides de Lewis tels que le trifluorure de bore. Ces procédés souffrent d'inconvénients majeurs : l'acide fluorhydrique en raison de sa toxicité et de sa forte volatilité ; l'acide sulfurique en raison d'une consommation importante du catalyseur nécessitant un retraitement onéreux. C'est pourquoi on a préconisé l'emploi de catalyseurs solides ou supportés sur des solides tels que les aluminosilicates ou les oxydes métalliques, par exemple la zircone traitée par de l'acide sulfurique.

Il a été proposé dans le brevet US-A-3.795.712 d'utiliser, pour catalyser les réactions d'alkylation de composés aromatiques par au moins une oléfine, d'alkylation aliphatique d'isobutane par au moins une oléfine, d'oligomérisation ou de polymérisation d'oléfines, des compositions catalytiques comprenant un acide de Lewis ou de Brönsted et une sulfone de formule R-SO₂-R', où R et R' sont chacun séparément un radical monovalent comportant de 1 à 8 atomes de carbone par molécule, ou forment ensemble un radical divalent comportant de 3 à 12 atomes de carbone, éventuellement dans un solvant hydrocarboné inerte, la concentration de l'acide étant comprise entre 10⁻⁵ mole par litre de sulfone et 5 moles par litre de sulfone, ce qui fait entre 0,00012 et 37 % poids d'acide dans le cas où l'acide est l'acide trifluorométhanesulfonique et où la sulfone est le sulfolane.

La demande de brevet PCT WO 93/00316 décrit des compositions catalytiques liquides pour l'alkylation aliphatique comprenant de 10 à 90% poids d'acide fluorhydrique ou sulfonique substitué par au moins un halogène et de 10 à 90% poids d'un solvant possédant un nombre donneur inférieur à 40 en l'absence d'ajout intentionnel d'halogénure de métal. Parmi de tels solvants dont la liste est nombreuse figurent le sulfolane ou tétraméthylène sulfone et le diméthylsulfoxyde.

Le catalyseur selon l'invention constitue un perfectionnement des compositions catalytiques liquides décrites dans US-A-3.795.712 et WO 93/00.316, utilisées en alkylation aliphatique .

La présente invention concerne un catalyseur comprenant un support poreux organique ou minéral et au moins une phase active comprenant au moins un acide choisi parmi les acides de formule R-SO₃H, où R est le fluor ou un groupement alkyl ou un groupement alkyl fluoré (partiellement ou totalement fluoré), R étant de préférence F ou CF₃, de façon encore plus préférée CF₃, et au moins un solvant aprotique organique faiblement basique, ledit support ayant été imprégné par ladite phase active, ledit catalyseur étant tel qu'il est constitué essentiellement de particules de diamètre moyen compris entre 0,1 et 150 µm (1 µm = 10⁻⁶ m), de préférence entre 5 et 110 µm et de manière encore plus préférée entre 5 et 80 µm, et tel que ledit support, avant son imprégnation par ladite phase active, possède un volume poreux total compris entre 0,5 et 6 cm³/g, de préférence entre 0,6 et 6 cm³/g et de façon encore plus préférée compris entre 1,5 et 6 cm³/g. De préférence, le catalyseur comprend un support poreux organique ou minéral et au moins une phase active comprenant de l'acide fluorosulfonique FSO₃H ou de l'acide trifluorométhanesulfonique CF₃SO₃H, et au moins un solvant aprotique organique faiblement basique. De façon encore plus préférée, le catalyseur comprend un support poreux organique ou minéral et au moins une phase active comprenant de l'acide trifluorométhanesulfonique CF₃SO₃H, et au moins un solvant aprotique organique faiblement basique.

Lorsque la silice est utilisée comme support, elle peut contenir des impuretés comme par exemple les. oxydes, les alcalins, les alcalino-terreux, les composés d'aluminium ou toute autre impureté connue de l'homme du métier, la quantité totale de ces impuretés n'excédant généralement pas 2 % en poids par rapport à la silice.

Le support poreux organique ou minéral, de préférence la silice, est généralement tel que, avant son imprégnation par la phase acide, sa surface spécifique est comprise entre 0,1 et 1 500 m²/g.

Le catalyseur comprend un support poreux organique ou minéral et au moins une phase active, imprégnée sur le support, comprenant au moins un acide choisi parmi les acides de formule R-SO₃H, où R est le fluor ou un groupement alkyl ou un groupement alkyl fluoré (partiellement ou totalement fluoré), R étant de préférence F ou CF₃, de façon encore plus préférée CF₃, et au moins un solvant aprotique faiblement basique. Ladite phase active comprend (en % poids) généralement entre 10 et 99 %, de préférence entre 30 et 99 %, et de manière encore plus préférée entre ' 40 et 99 % poids d'acide, et entre 1 et 90 %, de préférence entre 1 et 70 %, et de manière encore plus préférée entre 1 et 60 % poids du solvant aprotique organique faiblement basique.

L'acide utilisé possède généralement un titre, en % poids, compris entre 95 et 100 % et de préférence entre 98 % et 100 %, le complément à 100 % étant le plus souvent constitué d'eau.

Le solvant utilisé est un solvant organique aprotique faiblement basique. Il est généralement miscible à l'acide et non miscible à la phase hydrocarbonée. La basicité de ce solvant doit être la plus faible possible de manière à ne pas diminuer l'activité protonique de l'acide avec lequel il est en mélange. Le solvant est généralement choisi dans le groupe formé par le sulfolane, nom déposé du bioxyde de tétrahydrothiofène, le diméthylsulfoxyde (encore appelé diméthylsulfinone), le nitrométhane et les dioxannes (le dioxanne 1-2 et le dioxanne 1-4), et de préférence ledit solvant est choisi dans le groupe formé par le sulfolane, les dioxannes et le diméthylsulfoxyde et de façon encore plus préférée ledit solvant est le sulfolane.

Un des avantages du catalyseur utilisé selon l'invention, par rapport à l'acide sulfurique de titre 97 à 99 % poids actuellement utilisé dans les unités d'alkylation, est qu'il présente une acidité supérieure ou égale tout en présentant un caractère oxydant beaucoup plus faible, compte tenu du fait que l'acide compris dans ledit catalyseur possède un caractère oxydant beaucoup plus faible que l'acide sulfurique. D'autre part, un des avantages du catalyseur utilisé selon l'invention, par rapport à l'acide sulfurique, est la limitation du passage indésirable en solution hydrocarbonée de l'acide compris dans ledit catalyseur, sous forme de composés dénommés triflates d'alkyle dans le cas où l'acide est l'acide trifluorométhanesulfonique.

Un des avantages du catalyseur utilisé selon l'invention est aussi la qualité de l'alkylat obtenu, qui présente une teneur en soufre moindre que lors de l'utilisation d'acide sulfurique sur support.

Un autre des avantages du catalyseur utilisé selon l'invention est que ladite utilisation conduit donc à une réduction de la consommation de catalyseur et donc à une diminution des coûts opératoires des unités d'alkylation.

La phase active occupe généralement entre 80 et 100 % du volume poreux total du support, et de préférence entre 90 et 100 % dudit volume poreux.

Le procédé de préparation du catalyseur selon l'invention comprend généralement au moins deux étapes. Dans une première étape le support poreux organique ou minéral est calciné à une température supérieure à 50 °C, de préférence supérieure à 80 °C et de manière encore plus préférée comprise entre 150 et 600°C, par exemple égale à environ 500° C. La durée de cette étape de calcination est habituellement comprise entre 10 minutes et 50 heures, de préférence entre 15 minutes et 25 heures. La calcination est généralement effectuée en présence clair sec ou de mélange air sec/azote, de débit compris entre 0,001 et 10 l/h/g, de préférence entre 0,1 et 5 l/h/g. La deuxième étape consiste en l'imprégnation dudit support calciné par la phase active. Pour réaliser cette deuxième étape, on peut utiliser toutes les techniques connues de l'homme du métier. A ce procédé de préparation s'ajoute généralement une étape supplémentaire de préparation de la phase active, préalable à l'étape d'imprégnation.

L'invention concerne aussi l'utilisation du catalyseur selon l'invention en alkylation catalytique d'au moins une isoparaffine choisie dans le groupe formé par l'isobutane et l'isopentane (c'est-à-dire l'isobutane et ou l'isopentane : l'isobutane, ou l'isopentane, ou l'isobutane et l'isopentane) en présence d'au moins une oléfine comportant de 3 à 6 atomes de carbone par molécule.

Le catalyseur selon la présente invention est mis en oeuvre dans un procédé qui permet de réaliser dans les meilleures conditions la réaction d'alkylation de l'isoparaffine par les oléfines. En particulier, ladite réaction étant caractérisée par une forte exothermicité (environ 83,6 kJ/mol de butène transformé si l'oléfine est le butène et si l'isoparaffine est l'isobutane), la mise en oeuvre du catalyseur selon la présente invention permet d'obtenir une bonne homogénéité de température et de concentration en réactifs.

Dans le procédé d'alkylation de l'isoparaffine utilisant le catalyseur selon la présente invention, les conditions opératoires, et plus particulièrement la température et la pression, sont généralement choisies de façon à ce que le mélange constitué par l'isoparaffine, l'(les) oléfine(s) et les produits de la réaction soit liquide. De plus, il est important que le catalyseur soit immergé dans ledit liquide de façon à assurer un bon contact liquide-solide.

Le catalyseur selon l'invention est avantageusement mis en oeuvre dans la zone réactionnelle d'alkylation de l'isobutane et/ou de l'isopentane avec au moins une oléfine comprenant de 3 à 6 atomes de carbone par molécule, en phase liquide et en mélange avec l'isoparaffine et/ou un mélange d'isoparaffines. Le catalyseur selon l'invention peut être mis en oeuvre en lit expansé, en zone réactionnelle presque parfaitement agitée ou en lit circulant, et de préférence il est mis en oeuvre dans un procédé qui utilise une phase liquide continue, le catalyseur pouvant être utilisé sous forme de suspension selon les deux mises en oeuvre préférées décrites ci-après.

Une première mise en oeuvre préférée du catalyseur selon la présente invention est la zone réactionnelle à mélange presque parfait, c'est-à-dire à mélange parfait ou s'en rapprochant (cuve agitée ou Grignard), utilisant au moins un moyen d'agitation, par exemple au moins une hélice, de façon à obtenir une agitation suffisante du catalyseur en suspension dans la phase liquide hydrocarbonée, laquelle comprend généralement l'isoparaffine (isobutane et/ou isopentane), au moins une oléfine, éventuellement au moins un diluant inerte (par exemple le propane et le normal butane) et les produits de la réaction d'alkylation. La charge à convertir, constituée d'isobutane et/ou d'isopentane et d'au moins une oléfine, peut être par exemple introduite sous forme liquide en au moins un point au sein de la phase liquide hydrocarbonée présente dans la zone réactionnelle.

Une deuxième mise en oeuvre préférée du catalyseur selon la présente invention en suspension dans une phase hydrocarbonée est le lit mobile à co-courant c'est-à-dire le lit circulant. Dans cette mise en oeuvre le catalyseur en suspension dans la phase liquide hydrocarbonée, comprenant généralement l'isoparaffine (isobutane et/ou isopentane), au moins une oléfine, éventuellement au moins un diluant inerte (par exemple le propane ou le normal butane) et les produits de la réaction d'alkylation, circule de bas en haut dans la zone réactionnelle. L'ensemble constitué par la suspension de catalyseur dans la phase hydrocarbonée circule ensuite au travers d'au moins un échangeur de chaleur et d'au moins une pompe, avant d'être de nouveau introduit à l'entrée de la zone réactionnelle. La charge à convertir, constituée d'isobutane et/ou d'isopentane et d'au moins une oléfine, est introduite soit sous forme liquide, soit sous forme gazeuse en au moins un point de la zone réactionnelle.

Dans les deux types de mises en oeuvre décrites précédemment, l'isoparaffine (isobutane et/ou isopentane) n'ayant pas été convertie ou ayant été introduite en excès par rapport à la stoechiométrie de la réaction, est généralement recyclée après séparation de l'alkylat, soit par introduction directe dans la zone réactionnelle, soit par mélange avec la charge à convertir.

Le mélange isoparaffine(s)-oléfine(s) est généralement introduit dans la zone réactionnelle à une vitesse spatiale horaire, exprimée en poids d'oléfine introduite par unité de poids du catalyseur et par heure (pph) comprise entre 0,001 et 10 h⁻¹ et de préférence comprise entre 0,002 et 2 h⁻¹. Ledit mélange peut aussi être réalisé à l'intérieur de la zone réactionnelle. Dans tous les cas le mélange ainsi constitué est dans la zone réactionnelle dans des conditions de pression et de température telles que le mélange d'hydrocarbures reste liquide sur le catalyseur.

La température de réaction est généralement inférieure à +40°C, de préférence inférieure à + 15°C et de manière souvent plus préférée comprise entre +5°C et -5°C. La pression de la zone réactionnelle est généralement suffisante pour maintenir les hydrocarbures à l'état liquide dans ladite zone.

Afin de limiter les réactions secondaires, on utilise généralement un excès d'isoparaffine par rapport à l'oléfine. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 15.

Lorsque la nature du catalyseur et les conditions de réaction sont judicieusement choisies (en particulier la température), le catalyseur selon l'invention permet la production de produits d'alkylation d'au moins une isoparaffine par au moins une oléfine qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comprenant 70 à 98 % en moles de triméthylpentanes.

Un autre avantage du catalyseur selon la présente invention est la possibilité d'alkyler, à basse température, l'isobutane avec des mélanges d'oléfines comportant de 3 à 6 atomes de carbone par molécule, où la proportion d'oléfines comportant plus de 4 atomes de carbone par molécule est très importante.

Les exemples suivants illustrent l'invention sans toutefois en limiter la portée.

### EXEMPLE 1

### Préparation du catalyseur 1 selon l'invention

On prépare 100 g de phase active conforme à l'invention en mélangeant 80 g d'acide trifluorométhanesulfonique de titre 98 % poids et 20 g de sulfolane préalablement distillé sous vide. La phase acide ainsi préparée contient donc 20 % poids de sulfolane et 80 % poids d'acide trifluorométhanesulfonique de titre 98 % poids.

Puis on active 10 g de silice de surface spécifique égale à 383 m²/g, de volume poreux total égal à 2,6 cm³/g et de diamètre moyen des particules égal à 35 µm, par calcination sous air sec pendant 4 heures à 500°C. La silice ainsi activée est conservée sous argon. On procède alors à une imprégnation à sec et à l'abri de l'humidité, de 6,5 g de ladite silice par 26,5 g de phase active préparée selon le mode opératoire décrit ci-dessus.

Le solide ainsi obtenu, appelé catalyseur 1, possède une teneur pondérale en phase active égale à 80,3 % poids.

### Alkylation de l'isobutane par le butène-2 avxec le catalyseur 1

Le catalyseur 1 est utilisé pour alkyler l'isobutane par le butène-2 de façon à produire des paraffines ramifiées à hauts indices d'octane. On introduit 33 g du catalyseur 1, préparé ci-dessus, dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -10 °C.

200 ml d'isobutane sont alors ajoutés dans le réacteur contenant le catalyseur sous agitation, ledit réacteur étant immergé dans un bain froid à - 4 °C. On laisse sous agitation le système (catalyseur + isobutane) pendant une durée de 30 minutes afin d'homogénéiser la température.

Ensuite on ajoute en continu et de façon régulière 17,8 g de butène-2, pendant une durée totale de 5 heures, la température du réacteur étant maintenue à -1 °C pendant toute la durée de l'injection.

Après réaction, la phase hydrocarbure (alkylat + isobutane) est soutirée du réacteur, puis elle est analysé par chromatographie en phase vapeur. Sa composition pondérale est donnée dans le tableau 1. La conversion de l'oléfine est de 100 %.

### Exemple 2

### Préparation du catalyseur 2 comparatif

On utilise dans cet exemple de l'acide trifluorométhanesulfonique de titre 98% poids sans ajout.

On active 10 g de silice de surface spécifique égale à 383 m²/g et de volume poreux total égal à 2,6 cm³/g et de diamètre moyen des particules égal à 35 µm, par calcination sous air sec pendant 4 heures à 500°C. La silice ainsi activée est conservée sous argon. On procède alors à une imprégnation à sec et à l'abri de l'humidité, de 6,5 g de ladite silice par 26,5 g de phase active préparée selon le mode opératoire décrit ci-dessus.

Le solide ainsi obtenu, appelé catalyseur 2, possède une teneur pondérale en phase active égale à 80,3% poids.

### Alkylation de l'isobutane par le butène-2 avec le catalyseur 2

Le catalyseur 2 ainsi préparé est testé dans les mêmes conditions opératoires que celles décrites dans l'exemple 1. Les résultats catalytiques obtenus sont donnés dans le tableau 1. La conversion de l'oléfine est de 100%.

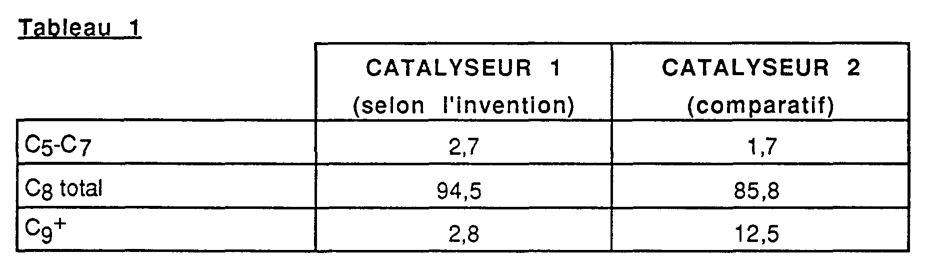

Le tableau 1 met en évidence l'effet positif de la présence d'un solvant aprotique organique faiblement basique dans la phase active comprenant l'acide trifluorométhanesulfonique, dans le cas présent ledit solvant étant le sulfolane. En effet, le catalyseur 1 conduit, pour des conditions de mise en oeuvre identiques, à une sélectivité en composés C₈ plus grande et à une sélectivité en composés lourds C₉⁺, composés indésirables, plus faible.

### Exemple 3

### Préparation du catalyseur 3 selon l'invention

On active 10 g de silice de volume poreux total égal à 2,6 cm³/g, de surface spécifique égale à 402 m²/g et de diamètre moyen des particules égal à 76 µm, par séchage à 150°C pendant 12 heures. La silice ainsi activée est conservée sous azote. On procède alors à une imprégnation à sec de 6,2 g de ladite silice par 25,2 g de la phase active préparée dans l'exemple 1.

Le solide ainsi obtenu, appelé catalyseur 3, possède une teneur pondérale en phase active égale à 80,25 % poids. Il est conservé à l'abri de l'humidité sous argon.

### Alkylation de l'isobutane par le butène avec le catalyseur 3

On introduit 31,4 g du catalyseur 3 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -10°C.

200 ml d'isobutane sont alors ajoutés dans le réacteur contenant le catalyseur sous agitation (hélice), ledit réacteur étant immergé dans un bain froid à -4°C. On laisse sous agitation le système (catalyseur 3 + isobutane) pendant une durée de 30 minutes afin d'homogénéiser la température.

On ajoute régulièrement 15,6 g de butène-2 par heure pendant une durée totale de 5 heures, la température du réacteur étant maintenue à -1 °C pendant toute la durée de l'injection.

Après réaction, la phase hydrocarbure (isobutane + alkylat) est soutirée du réacteur, et analysé par chromatographie en phase vapeur. Sa composition pondérale est donnée dans le tableau 2. La conversion de l'oléfine est de 100 %.

### Exemple 4

### Préparation du catalyseur 4 comparatif

On prépare 10 g de la même silice que celle utilisée dans l'exemple 3 et de façon identique à celle employée dans l'exemple 3. On procède alors à une imprégnation à sec de 6,2 g de ladite silice par 25,2 g de l'acide utilisé dans l'exemple 2.

Le solide ainsi obtenu, appelé catalyseur 4, et possède une teneur pondérale en phase acide égale à 80,25 % poids ; il est conservé à l'abri de l'humidité sous argon.

### Alkylation de l'isobutane par le butène-2 avec le catalyseur 4

On introduit 31,4 g du catalyseur 4 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -10°C. 200 ml d'isobutane sont alors ajoutés dans le réacteur contenant le catalyseur sous agitation (hélice), ledit réacteur étant immergé dans un bain froid à -4°C. On laisse sous agitation le système (catalyseur 4 + isobutane) pendant une durée de 30 minutes afin d'homogénéiser la température.

On ajoute régulièrement 15,6 g de butène-2 par heure pendant une durée totale de 5 heures, la température du réacteur étant maintenue à -1 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 2. La conversion de l'oléfine est de 100 %.

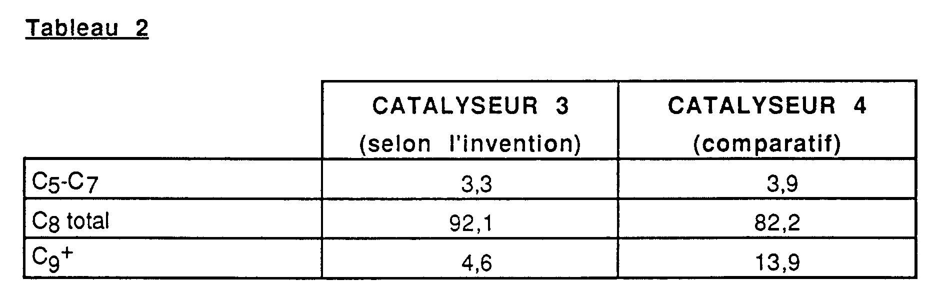

Le tableau 2 met en évidence l'effet positif de la présence d'un solvant aprotique organique faiblement basique dans la phase active comprenant l'acide trifluorométhanesulfonique, dans le cas présent ledit solvant étant le sulfolane. En effet, le catalyseur 3 conduit, pour des conditions de mise en oeuvre identiques, à une sélectivité en composés C₈ plus grande et à une sélectivité en composés lourds C₉⁺, composés indésirables, plus faible.

### Exemple 5

### Préparation du catalyseur 5 selon l'invention

On prépare 50 g de phase active conforme à l'invention en mélangeant 40 g d'acide trifluorométhanesulfonique de titre 100 % poids et 10 g de sulfolane préalablement distillé sous vide. Les 40 g d'acide trifluorométhanesulfonique de titre 100 % ont été obtenus par mélange de 30,45 g d'acide trifluorométhanesulfonique de titre 98 % poids et de 9,55 g de l'anhydride de l'acide trifluorométhanesulfonique (CF₃SO₂)₂O. La phase acide ainsi préparée contient donc 20 % poids de sulfolane et 80 % poids d'acide trifluorométhanesulfonique de titre 100 % poids.

Puis on prépare 10 g de la même silice que celle utilisée dans l'exemple 3 et de façon identique à la méthode employée dans l'exemple 3. On procède alors à une imprégnation à sec de 6,5 g de ladite silice par 26,1 g de la phase active préparée selon le mode opératoire décrit ci-dessus.

Le solide ainsi obtenu, appelé catalyseur 5, possède une teneur pondérale en phase acide égale à 80,1 % poids ; il est conservé à l'abri de l'humidité sous argon.

### Alkylation de l'isobutane par le butène-2 avec le catalyseur 5

On introduit 32,6 g du catalyseur 5 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -10°C.

200 ml d'isobutane sont alors ajoutés dans le réacteur contenant le catalyseur sous agitation (hélice), ledit réacteur étant immergé dans un bain froid à -4°C. On laisse sous agitation le système (catalyseur 5 + isobutane) pendant une durée de 30 minutes afin d'homogénéiser la température.

On ajoute régulièrement 19,6 g de butène-2 par heure pendant une durée totale de 5 heures, la température du réacteur étant maintenue à -1 °C pendant toute la durée de l'injection.

Après réaction, la phase hydrocarbure (alkylat + isobutane) est soutirée du réacteur, et est analysé par chromatographie en phase vapeur. Sa composition pondérale est donnée dans le tableau 3. La conversion de l'oléfine est de 100 %.

### Exemple 6

### Préparation du catalyseur 6 comparatif

50g d'acide trifluorométhanesulfonique de titre 100% sont obtenus par mélange de 38,06g d'acide trifluorométhanesulfonique de titre 98% poids et de 11,94g de l'anhydride de l'acide trifluorométhanesulfonique (CF₃SO₂)₂O. La phase acide ainsi préparée contient donc 100% poids d'acide trifluorométhanesulfonique (CF₃SO₃H) de titre 100% poids.

Puis on prépare 10 g de la même silice que celle utilisée dans les exemples 3 et 5 et en utilisant la même méthode que celle employée dans l'exemple 3. On procède alors à une imprégnation à sec de 6,5 g de ladite silice par 26,1 g de la phase active préparée selon le mode opératoire décrit ci-dessus.

Le solide ainsi obtenu, appelé catalyseur 6, et possède une teneur pondérale en phase acide égale à 80,1 % poids , il est conservé à l'abri de l'humidité sous argon.

### Alkylation de l'isobutane par le butène-2 avec le catalyseur 6

On introduit 32,6 g du catalyseur 6 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -10°C.

200 ml d'isobutane sont alors ajoutés dans le réacteur contenant le catalyseur sous agitation (hélice), ledit réacteur étant immergé dans un bain froid à -4°C. On laisse sous agitation le système (catalyseur 6 + isobutane) pendant une durée de 30 minutes afin d'homogénéiser la température.

On ajoute régulièrement 19,6 g de butène-2 par heure pendant une durée totale de 5 heures, la température du réacteur étant maintenue à -1 °C pendant toute la durée de l'injection.

Après réaction, la phase hydrocarbonée (alkylat + isobutane) est soutirée du réacteur, et est analysée par chromatographie en phase vapeur. Sa composition pondérale est donnée dans le tableau 3. La conversion de l'oléfine est de 100 %.

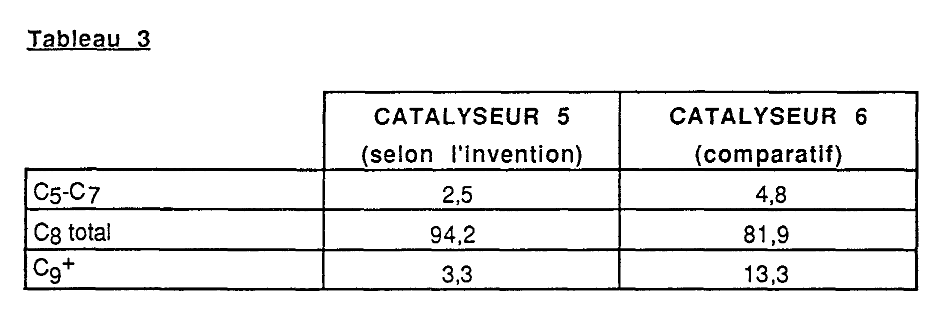

Le tableau 3 met en évidence l'effet positif de la présence d'un solvant aprotique organique faiblement basique dans la phase active comprenant l'acide trifluorométhanesulfonique, dans le cas présent ledit solvant étant le sulfolane. En effet, le catalyseur 5 conduit, pour des conditions de mise en oeuvre identiques, à une sélectivité en composés C₈ plus grande et à une sélectivité en composés lourds C₉⁺, composés indésirables, plus faible.

## Revendications

1. Catalyseur comprenant un support poreux organique ou minéral et au moins une phase active comprenant au moins un acide choisi parmi les acides de formule R-SO₃H, où R est le fluor ou un groupement alkyl ou un groupement alkyl fluoré, et au moins un solvant aprotique organique faiblement basique, ledit support ayant été imprégné par ladite phase active, ledit catalyseur étant tel qu'il est constitué essentiellement de particules de diamètre moyen compris entre 0,1 et 150 µm, et tel que ledit support, avant son imprégnation par ladite phase active, posséde un volume poreux total compris entre 0,5 et 6 cm³/g.

2. Catalyseur selon la revendication 1 tel que la phase active comprend entre 10 % et 99 % poids d'acide et entre 1 % et 90 % poids de solvant, et tel que l'acide posséde un titre compris entre 95 % et 100 % poids.

3. Catalyseur selon l'une des revendications 1 ou 2 tel que le solvant est choisi dans le groupe formé par le sulfolane, le diméthylsulfoxyde, le nitrométhane et les dioxannes.

4. Catalyseur selon l'une des revendications 1 à 3 tel que le solvant est choisi dans le groupe formé par le sulfolane, le diméthylsulfoxyde et les dioxannes.

5. Catalyseur selon l'une des revendications 1 à 4 tel que le solvant est le sulfolane.

6. Catalyseur selon l'une des revendications 1 à 5 tel que l'acide est l'acide fluorosulfonique ou l'acide trifluorométhanesulfonique.

7. Catalyseur selon l'une des revendications 1 à 6 tel que l'acide est l'acide trifluorométhanesulfonique.

8. Catalyseur selon l'une des revendications 1 à 7 tel que ledit support est la silice.

9. Utilisation du catalyseur selon l'une des revendications 1 à 8 en alkylation catalytique, dans une zone réactionnelle, d'au moins une isoparaffine choisie dans le groupe formé par l'isobutane et l'isopentane par au moins une oléfine comportant de 3 à 6 atomes de carbone par molécule, dans laquelle la température de réaction est inférieure à +40°C et la pression de la zone réactionnelle est suffisante pour maintenir les hydrocarbures à l'état liquide dans ladite zone.

10. Utilisation selon la revendication 9 dans laquelle le catalyseur est mis en oeuvre dans une zone réactionnelle à mélange presque parfait.

11. Utilisation selon la revendication 9 dans laquelle le catalyseur est mis en oeuvre en lit mobile à co-courant.

## Patentansprüche

1. Katalysator, welcher einen porösen organischen oder mineralischen Träger und mindestens eine aktive Phase umfasst, die mindestens eine unter den Säuren der Formel R-SO₃H gewählte Säure umfasst, wobei R Fluor oder eine Alkygruppe oder eine fuorierte Alkygruppe ist, und mindestens ein aprotisches, schwach basisches organisches Lösungsmittel, wobei der Träger mit dieser aktiven Phase imprägniert worden ist und wobei dieser Katalysator derart ist, dass er im wesentlichen aus Teilchen von einem mittleren Durchmesser zusammengesetzt ist, der zwischen 0,1 und 150 µm liegt, und derart, dass dieser Träger vor seiner Imprägnierung durch diese aktive Phase ein Gesamtporenvolumen hat, das zwischen 0,5 und 6 cm³/g liegt.

2. Katalysator nach Anspruch 1, derart, dass die aktive Phase zwischen 10 Gew.-% und 99 Gew.-% an Säure und zwischen 1 Gew.-% und 90 Gew.-% an Lösungsmittel umfasst und derart, dass die Säure einen Titer hat, der zwischen 95 Gew.-% und 100 Gew.-% liegt.

3. Katalysator nach einem der Ansprüche 1 oder 2, derart dass das Lösungsmittel in der durch Sulfolan, Dimethylsolfoxid, Nitromethan und die Dioxane gebildeten Gruppe gewählt ist.

4. Katalysator nach einem der Ansprüche 1 bis 3, derart dass das Lösungsmittel in der durch Sulfolan, Dimethylsolfoxid und die Dioxane gebildeten Gruppe gewählt ist.

5. Katalysator nach einem der Ansprüche 1 bis 4, derart dass das Lösungsmittel Sulfolan ist.

6. Katalysator nach einem der Ansprüche 1 bis 5, derart dass die Säure Fluorsulfon- oder Trifluormethansulfonsäure ist.

7. Katalysator nach einem der Ansprüche 1 bis 6, derart dass die Säure Trifluormethansulfonsäure ist.

8. Katalysator nach einem der Ansprüche 1 bis 7, derart dass der Träger Silica ist.

9. Verwendung eines Katalysators nach einem der Ansprüche 1 bis 8 bei der katalytischen Alkylierung von wenigstens einem aus der durch Isobutan und Isopentan gebildeten Gruppe gewählten Isoparaffin durch wenigstens ein Olefin, welches 3 bis 6 Kohlenstoffatome pro Molekül umfasst in einer Reaktionszone, in der die Reaktionstemperatur kleiner als +40°C und der Druck der Reaktionszone genügend ist, um die Kohlenwasserstoffe in flüssigem Zustand in dieser Zone zu halten.

10. Verwendung nach Anspruch 9, bei der der Katalysator in einer Reaktionszone bei fast perfekter Mischung eingesetzt wird.

11. Verwendung nach Anspruch 9, bei der der Katalysator im beweglichen Bett bei Gleichstrom eingesetzt wird.

## Claims

1. A catalyst comprising a porous organic or inorganic support and at least one active phase comprising at least one acid selected from acids with formula R-SO₃H, where R is fluorine or an alkyl group or a fluorinated alkyl group, and at least one weakly basic aprotic organic solvent, said support having been impregnated with the active phase, said catalyst being such that it is essentially constituted by particles with an average diameter in the range 0.1 µm to 150µ m, and being such that before impregnation with the active phase, said support has a total pore volume in the range 0.5 cm³/g to 6 cm³/g.

2. A catalyst according to claim 1, in which the active phase comprises 10% to 99% by weight of acid and 1% to 90% by weight of solvent, and in which the acid has a strength of 95% to 100%.

3. A catalyst according to claim 1 or claim 2, in which the solvent is selected from the group formed by sulpholane, dimethylsulphoxide, nitromethane and dioxanes.

4. A catalyst according to any one of claims 1 to 3, in which the solvent is selected from the group formed by sulpholane, dimethylsulphoxide, and dioxanes.

5. A catalyst according to any one of claims 1 to 4, in which the solvent is sulpholane.

6. A catalyst according to any one of claims 1 to 5, in which the acid is fluorosulphonic acid or trifluoromethanesulphonic acid.

7. A catalyst according to any one of claims 1 to 6, in which the acid is trifluoromethanesulphonic acid.

8. A catalyst according to any one of claims 1 to 7, in which said support is silica.

9. The use of a catalyst according to any one of claims 1 to 8 for the catalytic alkylation in a reaction zone of at least one isoparaffin selected from the group formed by isobutane and isopentane with at least one olefin containing 3 to 6 carbon atoms per molecule, in which the reaction temperature is below +40°C and the pressure in the reaction zone is sufficient to maintain the hydrocarbons in the liquid state in said zone.

10. Use according to claim 9, in which the catalyst is used in a near perfectly mixed reaction zone.

11. Use according to claim 9, in which the catalyst is used in a co-current mobile bed.
